# EUROPEAN PATENT APPLICATION

(11) **EP 3 173 015 A1**
(43) Date of publication of application: **31.05.2017**
(21) Application number: 16199982.6
(22) Date of filing: 15.04.2005
(51) Int. Cl.: A61B 5/00, G06F 19/00, A61B 5/021, A61B 5/07, A61B 5/20

(54) **PHYSIOLOGICAL EVENT HANDLING SYSTEM, METHOD, AND DEVICE; MONITORING DEVICE AND PROGRAM PRODUCT**

(30) Priority: 15.04.2004 US 825575
(62) Divisional of application: 05732310.7
(71) Applicant: Nokia Technologies OY, 02610 Espoo (FI)
(72) Inventor: HEINONEN, Tomi, 33610 Tampere (FI); KAUPPINEN, Pasi, 33610 Tampere (FI)
(74) Representative: Swindell & Pearson Limited

(57) **Abstract**

A system for handling a physiological event includes a monitoring device adapted to detect, sense, measure, simulate, intervent, or control one or more physiological parameters. The monitoring device is further adapted to transmit a signal including information corresponding at least to an identification of the monitoring device. The system also includes an event handling device adapted to receive signals from the monitoring device, including information corresponding to the identification of the monitoring device. The event handling device is further adapted to transmit a signal including information corresponding to the identification of the monitoring device.

## Description

### BACKGROUND OF THE INVENTION

The present invention relates generally to the field of health monitors, and particularly to systems and methods of handling physiological events that may be generated by a physiological monitor.

Health monitoring devices of various types have been used to monitor one or more physiological parameters for a patient. For example, electrocardiograms (ECG) and electroencephalograms (EEG) are capable of measuring electrical functions of the heart and brain, respectively. Also blood pressure, body weight and heart function, for example, can be measured, thereby producing physiological information. Typically, measured data are not collected and processed centrally, and storage requires manual interaction and/or a reading device specific for these independent systems.

In certain cases, the monitoring device may be mounted on the patient either permanently or on a temporary basis. For example, certain monitors may be implanted into the patient and may monitor certain physiological parameters on a continuous basis. The data from such devices may be recorded. Alternatively, as disclosed in U.S. Patent No. 6,428,475, an instantaneous value of the desired parameters may be displayed on a portable device.

### SUMMARY OF THE INVENTION

One embodiment of the invention relates to a method of handling a physiological event. The method comprises receiving a first signal from a monitor adapted to convey information relating to physiological parameters. The first signal can include information corresponding to the physiological parameters and an identification of the monitor. The method can also include transmitting a second signal to a network. The second signal can include at least information corresponding to the identification of the monitor.

In another embodiment, a system for handling a physiological event includes a monitoring device adapted to convey information relating to one or more physiological parameters. The monitoring device is further adapted to transmit a signal including information corresponding at least to an identification of the monitoring device. The system also includes an event handling device adapted to receive signals from the monitoring device, including information corresponding to the identification of the monitoring device. The event handling device is further adapted to transmit a signal including information corresponding to the identification of the monitoring device.

In another embodiment, the invention includes a monitoring device. The device includes a monitoring module for conveying information relating to one or more physiological parameters, and a transmitter adapted to transmit a signal. The signal includes information corresponding at least to an identification of the monitoring device.

In another embodiment, the invention includes an event handling device. The event handling device includes a receiving module adapted to receive signals from a monitor adapted to convey information relating to physiological parameters. The signals include information corresponding to the physiological parameters and an identification of the monitor. The device also includes a transmitting module adapted to transmit including at least information corresponding to the identification of the physiological monitor.

In still another embodiment of the invention, a program product comprises machine readable program code for causing a machine to perform the following steps: receiving a first signal from a monitor adapted to convey information relating to physiological parameters, and transmitting a second signal to a network. The first signal includes information corresponding to the physiological parameters and an identification of the monitor, and the second signal includes at least information corresponding to the identification of the monitor.

The monitors, monitoring modules, and/or monitor devices can be adapted to detect, sense, or measure the physiological parameters. They can also be adapted to stimulate, intervent, or control physiological functions affecting the physiological parameters. The physiological parameters can relate to any number of physiological functions such as, for example, heart and/or brain functions.

### BRIEF DESCRIPTION OF THE DRAWINGS

FIG. 1 is a diagrammatic illustration of one embodiment of an event handling system according to the invention; and
FIG. 2 is a schematic illustration of one embodiment of certain components of the event handling system of FIG. 1.

### DETAILED DESCRIPTION OF THE PREFERRED EMBODIMENTS

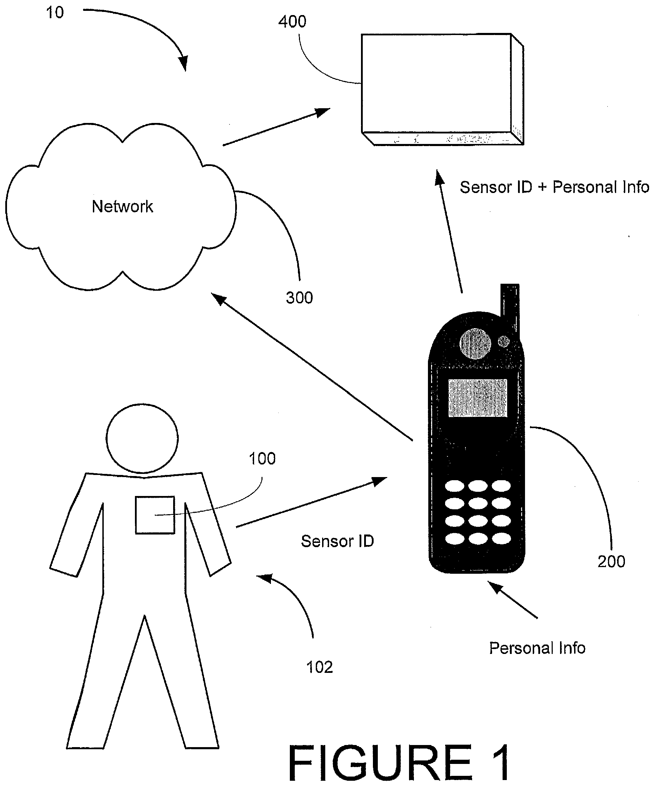

Referring to FIG. 1, an embodiment of an event handling system is diagrammatically illustrated. In the illustrated system 10, a monitoring device 100 is provided which could detect, measure or sense a predetermined set of physiological parameters for a patient 102. The monitoring device can also be adapted to stimulate, intervent, or control physiological functions affecting the physiological parameters. The monitoring device 100 may be externally connected to the patient 102. For example, the monitoring device 100 may be strapped around the chest or the wrist of the user to monitor heart function, for example. Such an arrangement may be used to measure the patient's heart rate, for example, and may be used to detect irregular heartbeat. Other examples of external monitoring devices 100 can include scales, blood pressure measurement devices, etc. In another embodiment, the monitoring device 100 may be implanted into the patient 102.

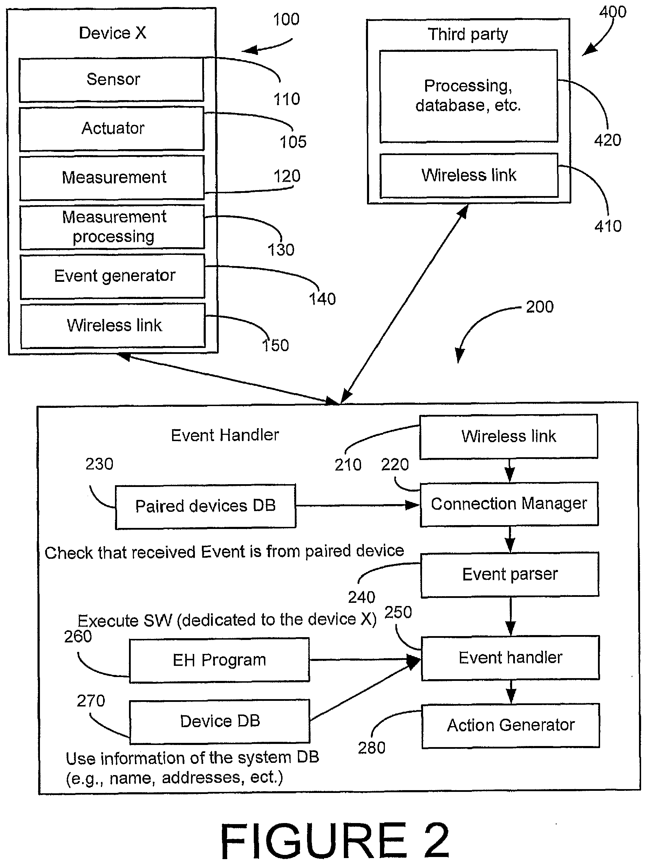

The monitoring device 100 may be provided with an internal power supply, such as a rechargeable battery. Other aspects of the monitoring device 100 are described below with reference to FIG. 2.

The monitoring device 100 is adapted to transmit signals to an event handling device 200. In the illustrated embodiment, the event handling device 200 is a wireless device, such as a cellular telephone. It will be understood by those skilled in the art that a variety of other devices may also be used as the event handling device 200. The communication between the monitoring device 100 and the event handling device 200 may be accomplished in a variety of ways. In a particular embodiment, the communication between the devices 100, 200 is accomplished through wireless signals. Wireless protocols, such as Bluetooth or other short range wireless communication technologies, may be used to facilitate the communication. Bluetooth is a standardized communication protocol. Products adapted to use this protocol are qualified for interoperability with all other Bluetooth products. Thus, the use of Bluetooth in the system 10 allows various components to communicate with each other, and eliminates the need for specialized equipment. Thus, a Bluetooth-qualified cellular telephone may be used to receive signals from the monitoring device 100. For additional information on the Bluetooth protocol, reference may be made to the Bluetooth Core Specification, Version 1.2.

The signals transmitted by the monitoring device 100 and received by the event handling device 200 include a variety of information. In particular, the signals contain information which uniquely identifies the monitoring device 100. In turn, this information definitively identifies the identity of the patient to whom the physiological monitoring device 100 belongs. Other information contained in the signals may include various parameters, such as data relating to heart function or brain function.

In one embodiment, the monitoring device transmits signals on a substantially continuous basis. For example, information relating to the patient's heart rate or blood pressure may be contained in signals that are transmitted by the monitoring device 100 at predetermined intervals, such as one second. In an alternate embodiment, the monitoring device 100 only transmits a signal when one or more physiological parameters satisfy a predetermined criteria. For example, a maximum measured heart rate, a maximum measured blood pressure level or a detected irregular heartbeat may cause the monitoring device 100 to transmit a signal.

The signal received by the event handling device 200 may be processed to determine the action to be taken by the event handling device 200. The processing of the signal may include verifying the source of the signal. As described below with reference to FIG. 2, the verification may include comparing the identification of the monitoring device 100 with identifications of various devices stored in a database. The database may be contained in the event handling device 200.

The processing may also include determining whether the information contained in the signal requires notification to a third party. For example, the signal may include information indicating that the patient requires immediate medical attention or the monitoring device 100 requires attention due to a malfunction or a low battery, for example. This information may take the form of a flag contained in the signal or raw physiological data which may be compared against thresholds stored in the event handling device 200.

When the event handling device 200 determines that a third party requires notification, a signal may be transmitted from the event handling device 200 to a communication network 300, through which a third party, such as a medical facility 400, is notified. The network 300 may be a wireless communication network, such as a cellular network. The signal transmitted by the event handling device 200 may include the information in the signal received by the event handling device 200 from the monitoring device 100. Additional information may be added by the event handling device 200, such as an identification of the event handling device 200. Further, the event handling device 200 may re-format the information to more readily convey the information to an operator at the medical facility 400, for example.

Thus, the third party receiving the signal from the event handling device 200, such as the medical facility 400, can quickly identify the event handling device 200 and evaluate the need for urgent medical attention. Since the signal also contains information corresponding to the identification of the monitoring device 100, the third party can, with certainty, identify the patient associated with the signal received from the event handling device 200.

In another embodiment of the invention, the monitoring device 100 can be configured, in certain situations, to broadcast a general emergency signal. In addition, software enabling minimal event handling capabilities can be included in various mobile devices such that these mobile devices are capable of receiving and processing the general emergency signal. In this situation, mobile devices within communication range of the monitoring device 100 can be configured to relay this message, through a communications network, to an emergency response facility. For example, if the patient 102 were to experience a cardiac arrest, the monitoring device 100 could detect this emergency situation and broadcast an emergency signal and all mobile devices equipped with the minimal event handling capabilities could detect and process the message.

More particularly, mobile devices, such as mobile telephones, may be configured to include at least event handling capabilities sufficient to recognize and act upon the emergency signal. The broadcast emergency signal could be of a very specific configuration such that the emergency response facility could recognize the location of the patient (or monitoring device 100) such as through the use of global positioning information. As such, the emergency response facility could dispatch a response team to the patient's location.

In other embodiments of the invention, a two-way communication channel can be opened up with the monitoring device 100 and used to maintain the monitoring device 100. As explained in more detail below, the monitoring device 100 can be configured to relay it's serial number or other identifying information. The system 10 can be configured to download software, such as device drivers or database information, to specific monitoring devices 100 based on predetermined maintenance schedules or on the specific functionality of the monitoring device 100. In addition, the two-way communication channel can be used to allow communication between monitoring devices 100.

FIG. 2 provides a schematic illustration of certain components of the system 10 in greater detail. As described above with reference to FIG. 1, the monitoring device 100 is adapted to transmit signals to the event handling device 200. A double arrow is shown in FIG. 2 to indicate that, in certain embodiments, instructions may be transmitted to the monitoring device 100 through the communication link with the event handling device 200. Similarly, a double arrow between the event handling device 200 and the third party 400 indicates a two-way communication link.

An embodiment of the monitoring device 100 will now be described with reference to FIG. 2. The monitoring device 100 may include one or more sensors 110 or measurement devices 120 to detect, sense or measure various physiological parameters. The monitoring device 100 may also include one or more actuators 105 for stimulating, interventing, and/or controlling physiological functions affecting the physiological parameters. Such actuators 105, sensors 110, and measurement devices 120 are well known to those skilled in the art.

A measurement processing module 130 is provided in the physiological monitoring device 100 to process the data from the sensors 110 or measurement devices 120. The measurement processing module 130 may determine whether an event has occurred requiring transmission of a signal. An event may be predefined as, for example, the expiration of an interval or one or more physiological parameters satisfying a predetermined criteria, such as maximum blood pressure. Events can originate based on a variety of detected stimuli. For example, events can originate from organs, such as the heart, brain, muscles, etc.) producing measurable electrical currents, potentials, or magnetic fields. The electrical or chemical properties of body fluids as affected by such factors as blood sugar, Cerebro spinal fluid conductivity, urine, etc, or mechanical movement or orientation of the body or body structures, such as organs, muscles, or body orientation, can also generate events. Even acoustic signals or thermal information about the body or body structures or fluids can generate events.

In one example, an event may be indicated when the measurement processing module 130 determines that the data indicates a fibrillation detected by the monitoring device 100, which may be a heart pacemaker. Thus, the measurement processing module 130 may conclude either that no event has occurred, in which case it continues to receive data from the sensors 110 or measurement devices 120, or that an event has occurred, in which case it delivers certain information to an event generator 140.

The event generator 140 assembles a message to be transmitted as a signal by the monitoring device 100. In one example, the message may include the following information: sender identification, sender type, time/date, physiological parameter, value of physiological parameter, and an end mark. The sender identification may be, for example, a serial number of the monitoring device 100 or an identifier of the patient to whom the monitoring device 100 belongs. In the case where the monitoring device 100 includes multiple sensors, a sensor ID may also be included. In the above-described fibrillation example, the message may appear as:
*SERIAL NO* + *PACEMAKER MODEL ABC* + *SENSOR ID* + *DATE* + *TIME +FATAL CARDIAC SYMPTOM+ ACUTE FIBRILLATION* + *END*
The message is then transmitted by the monitoring device 100 through a communication link, such as a wireless link 150.

The message from the monitoring device 100 is received by the event handling device 200. As noted above, in a particular embodiment, the communication between the monitoring device 100 and the event handling device 200 can be through a wireless link using the Bluetooth protocol. The signal containing the message is received by the event handling device 200 through a wireless link 210. A connection manager module 220 is provided to initially process the signal. The initial processing 220 includes determining whether the signal is received from a valid source. In this regard, the event handling device 200 may include a list or database 230 of paired devices with which the event handling device 200 can validly communicate. This can ensure that signals from other devices or similar devices belonging to nearby persons are not mistakenly processed.

Once the signal is determined to be from a valid paired device, the message in the signal is parsed by an event parser 240. The event parser 240 may be adapted to isolate certain information for processing by additional modules, while retaining other information for inclusion in a message to be transmitted by the event handling device 200. In this regard, the event parser 240 may isolate information relating to the physiological parameters for processing by an event handler module 250. The event handler module 250 executes a software algorithm 260 stored in the event handling device 200 to determine the appropriate action. The software algorithm 260 may be adapted to compare the physiological parameters to information stored in a device database 270, such as threshold levels for various actions.

In the above-described fibrillation example, the software algorithm may appear as follows:

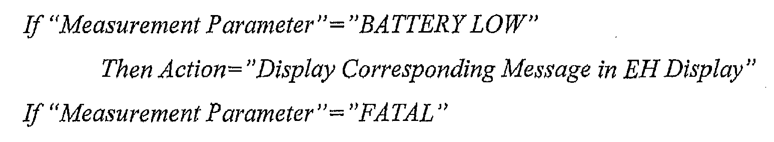

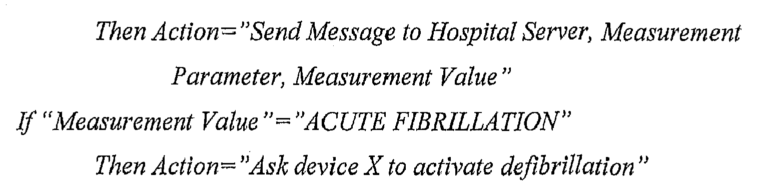

Thus, the event handler 250 can determine an appropriate action to be taken. The appropriate action may include transmitting a message indicating a medical emergency, malfunction of the monitoring device 100 or mere notification of the data relating to physiological parameters. In some cases, the appropriate action may be to take no action at all.

The event handler 250 can then transmit instruction to an action generator 280 corresponding to the action determined to be appropriate. In response, the action generator 280 generates a message to be transmitted to the third party 400. In this regard, the message generated by the action generator 280 may include all or part of the information received in the signal from the monitoring device 100 as well as additional information, such as identity of the event handling device 200. The generated message may also be formatted to be readily understandable by the third party 400. The formatted message is then sent by the action generator 280 to the wireless link 210 for transmission to the third party 400.

As noted above, the communication between the event handling device 200 and the third party 400 may be through a communication network, which may be a wireless network such as a cellular network. The signal from the event handling device 200 is received by a wireless link 410 in the facilities of the third party 400. The signal may then be processed by one or more modules 420 to be displayed, for example, to an operator, nurse or physician. Since the message contains information corresponding to the identification of the monitoring device 100, the patient's identity can be determined with great certainty.

The above-described system and associated components may have a variety of applications. Some exemplary applications are provided below.

### Cardiac Implant

The event handling device or a hospital may control the implanted monitoring device. If there was a malfunction in the implant, a notice may be given to the patient, who then has a possibility to respond to the situation. Routine visits to a physician for pacemaker control and maintenance may be reduced. Also, information may be available to the patient outside the hospital environment (e.g., when exercising there could be a notice to slow down in order to lower the heartrate.). If pacemaker detects changes in the heart, information would be available immediately, rather than waiting until the next routine check-up, which may be too late. A cardiac implant may also use defibrillation or another form of stimulation to affect or treat potential or actual heart problems.

### Epilepsy Implant

An epilepsy implant could recognize an impending or existing epileptic seizure. Epileptic seizures can occur without warning, thus severely restricting various aspects of a patient's life. Detection of an impending seizure may be delivered to the patient or a third party. An epilepsy implant may also be used to actively suppress an impending seizure.

### Drug Dosage Implant

Almost real-time information about the dosage used, changes in it and the status of the device may be made available to the patient or the third party.

### Blood Pressure Monitor

Measured values may be automatically stored in the memory of the event handling device, from which they can be accessed and displayed in an informative way (e.g. trend curves). Reliable and simple follow-up, the frequency of follow-up in hospital is thus dictated by actual needs. Physician may be immediately notified if necessary.

### Dialysis Monitor

Patients requiring dialysis operation periodically enter dialysis on a scheduled basis which, for conservative reasons, tends to be more often than needed. An implant can track a need for dialysis, and the data can be monitored by a medical facility. The medical facility can call the patient for treatment only when needed.

While particular embodiments of the present invention have been disclosed, it is to be understood that various different modifications and combinations are possible and are contemplated within the true spirit and scope of the appended claims. There is no intention, therefore, of limitations to the exact abstract and disclosure herein presented.

According to a first aspect of the present disclosure there is provided a method of handling an event, comprising: receiving a first signal from a monitor adapted to convey information relating to physiological parameters, the first signal including information corresponding to the physiological parameters and an identification of the monitor; and transmitting a second signal to a network, the second signal including at least information corresponding to the identification of the monitor.

The monitor may be an implant.

The monitor may be adapted to detect, sense, or measure the physiological parameters.

The monitor may be adapted to stimulate, intervent, or control physiological functions affecting the physiological, parameters.

The physiological parameters may relate to heart function.

The physiological parameters relate to brain function.

The first signal and the second signal may be wireless signals.

The network may be a wireless communication network.

The network may be a cellular network.

The method may further comprise: processing the first signal prior to transmitting the second signal.

The processing may further comprise: verifying a source of the first signal; identifying an event associated with the first signal and related to the physiological parameters; and determining a target for the second signal.

According to a second aspect of the present disclosure there is provided a system for handling an event, comprising: a monitoring device adapted to convey information relating to one or more physiological parameters, the monitoring device being further adapted to transmit a signal, the signal including information corresponding at least to an identification of the monitoring device; and an event handling device adapted to receive signals from the monitoring device including information corresponding to the identification of the monitoring device, the event handling device being further adapted to transmit a signal including information corresponding to the identification of the monitoring device.

The monitoring device may be implanted in a human body.

The monitoring device may be adapted to detect, sense, or measure the physiological parameters.

The monitoring device may be adapted to stimulate, intervent, or control physiological functions affecting the physiological parameters.

The physiological parameters may relate to heart function.

The physiological parameters may relate to brain function.

The physiological monitoring device may be adapted to transmit wireless signals.

The monitoring device may be adapted to transmit a signal when one or more physiological parameters satisfies a predetermined criteria.

The monitoring device may be adapted to transmit signals on a substantially continuous basis.

The event handling device may be adapted to transmit signals when one or more physiological parameters satisfies a predetermined criteria.

The event handling device may be adapted to transmit wireless signals to a network.

The event handling device may comprise: a data processing module adapted to verify a source of signals received, the data processing module being further adapted to identify an event associated with received signals and to determine a target for transmitted signals.

According to a third aspect of the present disclosure there is provided a physiological monitoring device, comprising: a monitoring module for conveying information relating to physiological parameters; and a transmitter adapted to transmit a signal, the signal including information corresponding at least to an identification of said monitoring module.

The monitoring module may be implanted in a human body.

The monitoring module may be adapted to detect, sense, or measure the physiological parameters.

The monitoring module may be adapted to stimulate, intervent, or control physiological functions affecting the physiological parameters.

The physiological parameters may relate to heart function.

The physiological parameters may relate to brain function.

The transmitter may be adapted to transmit wireless signals.

The transmitter may be adapted to transmit the signal when one or more physiological parameters satisfies a predetermined criteria.

The transmitter may be adapted to transmit the signal on a substantially continuous basis.

According to a fourth aspect of the present disclosure there is provided an event handling device, comprising: a receiving module adapted to receive signals from a monitor adapted to convey information relating to physiological parameters, the signals including information corresponding to the physiological parameters and an identification of the monitor; and a transmitting module adapted to transmit signals including at least information corresponding to the identification of the monitor.

The monitor may be adapted to detect, sense, or measure the physiological parameters.

The monitor may be adapted to stimulate, intervent, or control physiological functions affecting the physiological parameters.

The transmitting module may be adapted to transmit signals when one or more physiological parameters satisfies a predetermined criteria.

The transmitting module may be adapted to transmit wireless signals to a network.

The device may further comprise: a data processing module adapted to verify a source of signals received by the receiving module, the data processing module being further adapted to identify an event associated with the signals received by the receiving module and to determine a target for signals transmitted by the transmitting module.

According to a fifth aspect of the present disclosure there is provided a program product, comprising machine readable program code for causing a machine to perform the following steps: receiving a first signal from a monitor adapted to convey information related to physiological parameters, the first signal including information corresponding to the physiological parameters and an identification of the monitor; and transmitting a second signal to a network, the second signal including at least information corresponding to the identification of the monitor.

The invention is as set out in the following claims.

## Claims

1. A method comprising:
receiving, at a mobile wireless event handling device (200), a first signal via a wireless communication link in a first network, from a monitoring device (100) configured to convey information relating to physiological parameters, the first signal including information corresponding to an identification of the monitoring device (100);
receiving, at the mobile wireless event handling device (200), a general emergency signal from the monitoring device (100); and
in response to said receiving the general emergency signal, determining to transmit, at the mobile wireless event handling device (200), to a target, a second signal via a second network, the second signal including at least the general emergency signal and information corresponding to the identification of the monitoring device (100).

2. The method of any preceding claim, wherein the first signal and the second signal are wireless signals.

3. The method of any preceding claim, wherein the first network and the second network are different networks.

4. The method of any preceding claim, further comprising:
verifying a source of the first signal;
identifying an event associated with the first signal and related to the physiological parameters; and
determining a target (400) for the second signal.

5. The method of any preceding claim, further comprising, when the mobile wireless event handling device (200) determines that a third party requires notification, transmitting from the mobile wireless event handling device (200) to the third party.

6. The method of any preceding claim, further comprising, when one or more physiological parameters satisfy a predetermined criteria, transmitting signals.

7. The method of any preceding claim, wherein the second signal further includes identification of the mobile wireless event handling device (200).

8. The method of any preceding claim, wherein the monitoring device (100) is an implant.

9. The method of any preceding claim, wherein the monitoring device (100) is configured to:
detect, sense, or measure the physiological parameters; and/or
stimulate, intervent, or control physiological functions affecting the physiological parameters.

10. The method of any preceding claim, wherein the general broadcast emergency signal is received by all mobile wireless event handling devices (200) within communication range of the monitoring device (100).

11. The method of claim 10, wherein the mobile wireless event handling devices (200) are equipped with at least minimal event handling capabilities for receiving the general broadcast emergency signal.

12. A mobile wireless event handling device (200), comprising:
a receiving module configured to receive:
a first signal via a wireless communication link in a first network, from a monitoring device (100) configured to convey information relating to physiological parameters, the first signal including information corresponding to an identification of the monitoring device (100), and
a general emergency signal from the monitoring device (100); and
a data processing module configured to, in response to said receiving the general emergency signal, determine to transmit to a target, a second signal via a second network, the second signal including at least the general emergency signal and information corresponding to the identification of the monitoring device (100).

13. The device of claim 12, further comprising a transmitting module configured to transmit the second signal.

14. A system for handling an event, comprising:
the mobile wireless event handling device (200) of any of claims 1 to 11; and
a monitoring device (100) configured to send the first signal and the general emergency signal to the mobile wireless event handling device (200).

15. A computer program that, when performed by at least one processor, causes the method as claimed in any one or more of claims 1 to 11 to be performed.
